# EUROPÄISCHE PATENTSCHRIFT

(11) **EP 3 554 432 B1**
(45) Veröffentlichungstag und Bekanntmachung des Hinweises auf die Patenterteilung: **18.11.2020**
(21) Anmeldenummer: 17818091.5
(22) Anmeldetag: 11.12.2017
(51) Int. Cl.: A61F 5/442, A61F 5/451

(54) **VORRICHTUNG ZUR HYGIENISCHEN ENTSORGUNG VON HUMANEN AUSSCHEIDUNGSPRODUKTEN**
DEVICE FOR HYGENIC DISPOSAL OF HUMAN EXCRETIONS
DISPOSITIF POUR L'ÉVACUATION HYGIÉNIQUE DES EXCRÉTIONS HUMAINES

(30) Priorität: 14.12.2016 AT 511372016
(43) Veröffentlichungstag der Anmeldung: 23.10.2019
(73) Patentinhaber: pro aqua Diamantelektroden Produktion GmbH & Co KG, 8712 Niklasdorf (AT)
(72) Erfinder: SCHELCH, Michael, 8600 Oberaich (AT); STABER, Wolfgang, 8600 Bruck an der Mur (AT); HERMANN, Robert, 8600 Oberaich (AT); WESNER, Wolfgang, 1220 Wien (AT)
(74) Vertreter: Vinazzer, Edith
(86) Internationale Anmeldenummer: PCT/EP2017/082287
(87) Internationale Veröffentlichungsnummer: WO 2018/108842

(56) Entgegenhaltungen:
- WO-A1-00/69377
- JP-A- S61 342
- US-A1- 2004 143 229
- US-B1- 6 641 567

## Beschreibung

Die Erfindung betrifft eine Vorrichtung zur hygienischen Entsorgung von humanen Ausscheidungsprodukten, welche eine Hose aus einem gas- und flüssigkeitsdichten und elastischen Material mit einem Bauchbund und Beinbünden aufweist, wobei die Hose mit zumindest einem mit einer Absaugeinrichtung verbindbaren Absaugschlauch versehen ist oder mit einem solchen verbindbar ist.

Personen, die aufgrund ihres Entwicklungsstadiums, ihrer körperlichen Verfassung oder aus anderen Gründen, wie beispielsweise dem Tragen eines Schutzanzuges, über einen längeren Zeitraum nicht in der Lage sind, Toiletten zu benützen oder ihre Notdurft im Freien zu verrichten, sind bislang auf das Tragen von Windeln angewiesen. Windeln stellen für einen bestimmten, begrenzten Zeitraum ein körpernahes Speichersystem für Exkremente und gegebenenfalls auch Menstruationsblut dar. Die in Windeln verwendeten Sorptionsmedien haben zwar bereits einen sehr hohen Entwicklungsstand erreicht, ist jedoch ein zeitnahes Wechseln nicht möglich oder wird es nicht durchgeführt, so verbleibt unter Umständen über einen relativ langen Zeitraum ein Kontakt der Exkremente mit den in der Windel enthaltenen bzw. den sich entwickelnden Bakterien zur Haut des Trägers bestehen. Insbesondere für Erwachsene ist die Benützung von Windeln mit sehr unangenehmen Wahrnehmungen beim Tragen verbunden. Auch das Wechseln der Windeln wird, insbesondere von Pflegepersonal, als unangenehm empfunden.

Eine Vorrichtung der eingangs genannten Art ist beispielsweise aus der US 2004/0143229 A1 bekannt. Diese als Hose gestaltete Vorrichtung ist ausschließlich zum Sammeln und Ableiten von flüssigen Ausscheidungsprodukten geeignet und im Tragekomfort verbesserungswürdig.

Der Erfindung liegt die Aufgabe zugrunde, eine am Körper zu tragende Vorrichtung zur hygienischen Entsorgung von menschlichen Ausscheidungsprodukten, wie Exkrementen und Menstruationsblut, zur Verfügung zu stellen, die über einen langen Zeitraum getragen werden kann, nicht mit den Nachteilen von Windeln verbunden ist und einen hohen Tragekomfort gewährleistet.

Gelöst wird die gestellte Aufgabe erfindungsgemäß dadurch, dass die Hose eine mit offenporigem Schaumstoff und/oder mit elastischen Noppen bedeckte Innenseite sowie eine Einrichtung zum Einbringen von Flüssigkeit aufweist und wobei der Bauchbund und die Beinbünde innenseitige Einsätze aus offenporigem Schaumstoff aufweisen.

Die Noppen oder der Schaumstoff an der Innenseite der Hose schaffen zwischen der Haut und der Hose einen definierten und durchströmbaren Raum. Dieser macht es möglich, durch den offenporigen Schaumstoff am Bauchbund und den Beinbünden in den Innenraum Luft oder ein anderes Gas derart einzubringen, dass aus dem Inneren keine Flüssigkeit nach außen gelangt und dass über den Absaugschlauch eine Abführung von Exkrementen sowie das Absaugen der Luft/des Gases durchgeführt werden kann. Dabei kann über die Einrichtung zum Einbringen von Flüssigkeit sowohl Wasser als auch eine Reinigungsflüssigkeit in das Innere eingeleitet werden, um den Abtransport von Ausscheidungsprodukten zu unterstützen und um eine Reinigung und Desinfektion der Haut durchzuführen. Der Luft- bzw. Gasstrom sorgt anschließend für das Trocknen der Haut.

Die Vorrichtung eignet sich vor allem zur Benützung von Personen, die insbesondere über einen längeren Zeitraum nicht in der Lage sind, Toiletten zu benutzen und ansonsten auf das Tragen von Windeln mit allen Nachteilen angewiesen wären.

Bei einer bevorzugten Ausführungsform der Vorrichtung weist die Einrichtung zum Einleiten von Flüssigkeiten Ringdüsen auf, die in oder auf den Einsätzen aus offenporigem Schaumstoff positioniert sind. Dabei befindet sich bei jedem Einsatz eine umlaufende Ringdüse, die für ein optimales Einbringen von Flüssigkeiten in den Innenraum der Hose zur Unterstützung der Ableitung von Ausscheidungsprodukten sowie zur Desinfektion und Reinigung des Innenraums und der Haut des Trägers sorgt.

Bei einer weiteren bevorzugten Ausführung verfügt die Vorrichtung auch über eine Einrichtung zum Einleiten von Luft oder einem Gas, wobei diese Einrichtung weitere Ringdüsen im Bereich des Bauchbundes und der Beinbünde umfasst, die ausserhalb der zum Einleiten von Flüssigkeiten vorgesehenen Ringdüsen positioniert sind. Eine solche Einrichtung wird vor allem dann vorgesehen, wenn eine ausreichende Luftzufuhr über die Umgebungsluft nicht gewährleistet ist, was beispielsweise bei bettlägerigen Personen der Fall sein kann oder bei Personen, bei welchen die erfindungsgemäße Vorrichtung Bestandteil eines Schutzanzuges ist, welcher über einen längeren Zeitraum getragen wird.

Die Zuführung von Flüssigkeit sowie von Luft oder Gas zu den betreffenden Ringdüsen kann auf einfache Weise über jeweils zumindest einen Zuführschlauch und gegebenenfalls zumindest einen Verteilungsschlauch erfolgen. Verteilungsschläuche sind vor allem zur Verbindung der einzelnen Ringdüsen untereinander vorteilhaft und können an der Außen- oder der Innenseite der Hose entlanggeführt werden.

Die aus offenporigem Schaumstoff bestehenden Einsätze weisen vorzugsweise eine Breite von 1,0 cm bis 5,0 cm auf, ihre Dicke kann 0,1 cm bis 5,0 cm, insbesondere 0,5 cm bis 1,0 cm, betragen. Der für die Einsätze und der ggf. für die Innenseite vorgesehene offenporige Schaumstoff besteht vorzugsweise aus einem haltbaren und hautfreundlichen Material, insbesondere aus Polypropylen oder Polyethylen.

Die an der Innenseite der Hose vorgesehenen Noppen bestehen bei einer bevorzugten Ausführung aus dem Material der Hose und werden vorzugsweise bei der Herstellung der Hose mitgebildet. Die Noppen können eine Höhe von 0,5 cm bis 3,0 cm, insbesondere bis 1,0 cm, aufweisen.

Die Ausgestaltung der Noppen selbst kann auf unterschiedliche Weise erfolgen. Bevorzugt ist eine Ausführung, bei der die Noppen langgestreckte Elemente mit abgerundeter Spitze, ähnlich zu Patronenhülsen, sind, oder kegelförmig, halbkugelförmig und dergleichen ausgeführt sind.

Bevorzugt ist ferner eine Ausführung, bei der die Hose, ggf. gemeinsam mit den Noppen, aus einem Elastomer, insbesondere aus einem Gummimaterial oder aus Kunststoff, oder aus Silikon besteht.

Insbesondere bei einer vorgesehenen Verwendung der Vorrichtung in Schutzanzügen ist es vorteilhaft, wenn die Vorrichtung eine Trenneinrichtung zum Trennen der im abgesaugten Gemisch enthaltenen Gase, Flüssigkeiten und Feststoffe sowie auch einen Speicher zur Aufnahme der abgetrennten Flüssigkeit und/oder der abgetrennten Feststoffe aufweist. Bei einer Verwendung in einem Schutzanzug ist es ferner vorteilhaft, wenn die erfindungsgemäße Vorrichtung eine Einrichtung zum Aufbereiten des abgetrennten Gases zu dessen Wiederverwendung aufweist.

Weitere Merkmale, Vorteile und Einzelheiten der Erfindung werden nun anhand der schematischen Zeichnung, die ein Ausführungsbeispiel darstellt, näher beschrieben. Dabei zeigen
Fig. 1 eine Außenansicht einer zu einer erfindungsgemäßen Vorrichtung gehörende Hose und
Fig. 2 eine Schrägansicht der Hose mit einem Absaugschlauch jedoch ohne weitere Schläuche bzw. Zuführungen.

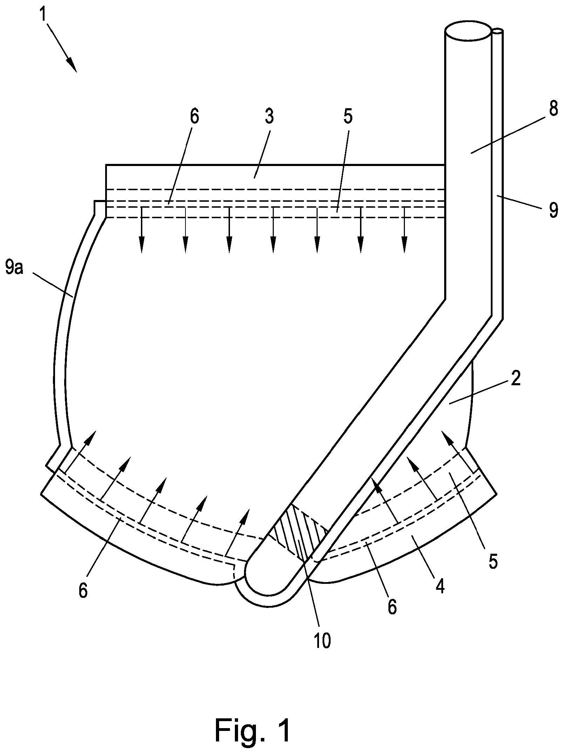

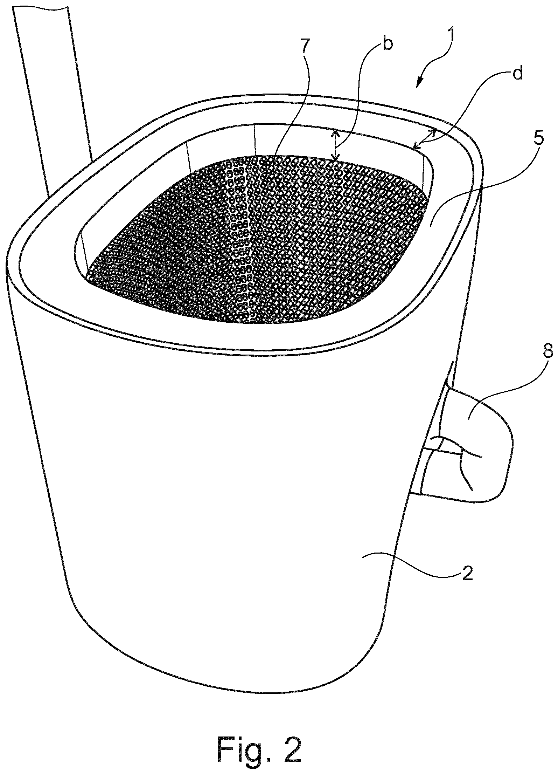

Die erfindungsgemäße Vorrichtung gemäß Fig. 1 und 2 weist eine Hose 1 auf, die entweder beinfrei (Fig. 1) ausgeführt ist oder nur mit kurzen Beinen versehen ist. Die Hose 1 weist eine Außenhülle 2 auf, welche aus einem gas- und flüssigkeitsdichten sowie elastischen und hautfreundlichen Material, vorzugsweise aus einem Elastomer, insbesondere aus einem Gummimaterial oder aus Kunststoff, oder aus Silikon besteht. Unter Gummimaterial wird dabei jede geeignete vulkanisierte Kautschukmischung verstanden, wobei als Kautschuk/Kautschuke insbesondere Naturkautschuk und/oder Polyisopren verwendet sein können. Die Hose 1 weist ferner einen Bauchbund 3 und zwei Beinbünde 4, die jeweils von einem an der Innenseite der Außenhülle 2 ringförmig umlaufenden Einsatz 5 (Fig. 2) aus einem offenporigen Kunststoffschaum, beispielsweise aus Polypropylen oder Polyethylen, gebildet sind. Die Breite b der Einsätze 5 beträgt vorzugsweise 1,0 cm bis 5,0 cm, die Dicke d der Einsätze 5 vorzugsweise 0,1 cm bis 3,0 cm, vorzugsweise bis 1,0 cm. Durch die Einsätze 5 hindurch kann Luft oder ein anderes Gas in das Innere der Hose 1 eingeströmt werden, so dass durch den offenporigen Schaum keine Flüssigkeit nach außen dringen kann.

Wie insbesondere Fig. 2 zeigt ist die Innenseite der Außenhülle 2 ist mit einer Vielzahl von Noppen 7 bedeckt, die vorzugsweise aus dem gas- und flüssigkeitsdichten sowie elastischen und hautfreundlichen Material der Außenhülle 2 bestehen. Die Noppen 7 sind insbesondere kegelförmig mit abgerundeter Spitze und weisen beispielsweise eine Höhe von 0,5 cm bis 3,0 cm auf, wobei sämtliche Noppen 7 gleich ausgeführt sein können. In bestimmten Bereichen der Innenseite der Außenhülle 2 können jedoch niedrigere oder höhere Noppen 7 als im Großteil der Innenseite vorgesehen sein. Die Noppen 7 können ferner einen beliebigen, mit abgerundeter Spitze versehenen Querschnitt aufweisen und können derart angeordnet sein, dass sie unmittelbar aneinander anschließen oder unter gegenseitigen Abständen von einigen Millimetern positioniert sind, wobei auch diesbezüglich in bestimmten Bereichen der Innenseite der Außenhülle 2 eine größere oder auch eine geringere Dichte an Noppen 7 als im Großteil der Innenseite vorgesehen sein kann. Durch die Noppen 7 wird zwischen der Haut des Trägers und der Innenseite der Außenhülle 2 der Hose 1 ein im Volumen definierter, durchströmbarer Raum geschaffen, wobei die Anordnung der Noppen 7 ferner derart ist, dass über den gesamten Innenraum der Hose 1 eine möglichst gleichmäßige Durchströmung mit Luft/Gas möglich ist.

Alternativ zu den Noppen kann die Innenseite mit offenporigem Schaumstoff bedeckt sein, analog zum Schaumstoff der Einsätze 5 und in einer Dicke, die der Dicke der Einsätze 5 entspricht. Die Schaumstoffschicht kann ferner an die Körperkonturen des Trägers angepasst sein.

In den Einsätzen 5 aus offenporigem Schaumstoff ist je eine Ringdüse 6 positioniert, deren vorgesehenen Öffnungen in Richtung des durchströmbaren Innenraums der Hose 1 weisen, wie es in Fig. 1 mit Pfeilen angedeutet ist. Ein Zuführschlauch 9 und zumindest ein Verteilungsschlauch 9a sorgen für ein Beschicken sämtlicher Ringdüsen 6 mit Flüssigkeit, wie noch beschrieben wird. Die Hose 1 ist ferner mit einem Absaugschlauch 8 verbunden, welcher sich vorzugsweise in räumlicher Nähe zum Anus befindet und an der Vorderseite der Hose 1 verläuft. In der Nähe der Absaugstelle des Absaugschlauches 8 befindet sich innerhalb der Absaugschlauches 8 eine Zerkleinerungseinrichtung 10 für Fäkalien, so dass diese leichter abtransportieren werden können. In einer einfachen Ausführung besteht die Zerkleinerungseinrichtung 10 beispielsweise aus zwei kreuzweise angeordneten Schneidblättern. Der Absaugschlauch 8 wird bei der Benützung der Hose 1 an eine Absaugeinrichtung angeschlossen, die auch für die Abführung der durch die Einsätze 5 ins Innere der Hose 1 strömenden Luft sorgt. Bei einer Benützung der Hose 1 in Bereichen mit Umgebungsluft, etwa in Krankenhäusern oder Pflegeheimen, kann die zugeführte Luft aus der Umgebungsluft kommen, in vielen Fällen, etwa bei der Verwendung in einem Schutzanzug, ist eine gesonderte Luft/Gasversorgungseinrichtung vorgesehen. Die Luft/Gaszufuhr kann dabei auch über weitere Ringdüsen erfolgen, die im Bereich des Bauchbundes und der Beinbünde jeweils außerhalb der Ringdüsen 6 verlaufen. Dabei wird die Außenhülle 2 randseitig über die Einsätze 5 geführt, um einen dichten Abschluss der Hose 1 zur Haut des Träger zu erreichen. Die weiteren Ringdüsen können zwischen dem über die Außenseiten der Einsätze verlaufenden Material der Außenhülle und dem jeweiligen Einsatz 5 eingebracht sein und im Schaumstoffmaterial eingebettet sein.

Mittels der erfindungsgemäßen Hose 1 kann für eine zeitnahe Abführung von Ausscheidungsprodukten gesorgt werden, indem bei Bedarf eine Absaugung über den Absaugschlauch 8 durchgeführt wird. Unmittelbar vor der Ausscheidung wird die Absaugung in Betrieb genommen, um eine gleichmäßige Durchströmung des Innenraumes mit Luft sicherzustellen. Gleichzeitig oder unmittelbar nach Beginn der Absaugung wird Reinigungsflüssigkeit über die Ringdüsen 6 in das Innere der Hose 1 geleitet und ebenfalls abgesaugt. Als Reinigungsflüssigkeit eignet sich vorzugsweise Wasser oder eine basische Lösung zur Desinfektion und Reinigung. Besonders bevorzugt ist eine alkalische Lösung mit einem pH von 11,0 bis 12,0. Der Reinigungsflüssigkeit können Reinigungsmittel wie Tenside oder Desinfektionsmittel zugesetzt sein. Die die Hose 1 durchströmende Luft bzw. das die Hose 1 durchströmende Gas hat sicherzustellen, dass sämtliche Flüssigkeiten sicher und ausschließlich in die vorgesehene Richtung, Richtung Absaugschlauch 8 transportiert werden.

Das aus der Hose 1 abgesaugte Gemisch aus Luft, Reinigungsflüssigkeit und Fäkalien wird vorzugsweise zunächst einer Stofftrennung unterzogen, wobei die Flüssigkeit mit den suspendierten oder gelösten Feststoffen von der Luft getrennt wird. Eine Aufbereitung der Luft bzw. des Gases kann mit einem der bekannten Reinigungsverfahren erfolgen. Die Fäkalflüssigkeit wird gegebenenfalls nach Zwischenspeicherung in einem Behälter über die Kanalisation entsorgt oder alternativ analog zur Reinigungsflüssigkeit aufbereitet.

Die vorgesehene Absaugung der Hose 1 kann von einer ereignisgesteuerten Regelung aus, zum Beispiel durch Auslösung durch den Träger, erfolgen oder etwa bei hoch pflegebedürftigen Personen, in einem permanenten Betrieb erfolgen. In Pflegeheimen oder anderen Einrichtungen, bei denen eine hohe Nutzungsdichte an Hosen zu erwarten ist, kann die Absaugung vorzugsweise über ein zentrales Absaugsystem durchgeführt werden, an welches die einzelnen Hosen, zum Beispiel über Schlauch-Kupplungssysteme, angeschlossen werden.

Ein Abführen von Ausscheidungsprodukten kann zum Beispiel folgende aufeinanderfolgende Schritte umfassen:
- Einschalten der Absaugung zum Einleiten von Luft/Gas
- Zufügen von sauberem Wasser zum Luftstrom über die Ringdüsen 6, um einen sicheren Abtransport der Fäkalien zu ermöglichen,
- Zufügen von Reinigungsflüssigkeit zum Luftstrom über die Ringdüsen 6, um eine Reinigung und Desinfektion der Haut durchzuführen,
- Zufügen von sauberem Wasser zum permanenten Luftstrom über die Ringdüsen 6 zum Abspülen des Reinigungsmittels,
- abschließendes Zuführen von Luft ohne Flüssigkeit zum Trocknen der Haut.

Bei einem Permanentbetrieb der Hose 1 können diese Schritte in ständiger Abfolge durchgeführt werden.

Wird die Vorrichtung von Personen in Schutzanzügen verwendet, können sämtliche zu ihrem Betrieb erforderlichen Aggregate im Schutzanzug selbst untergebracht werden.

### Bezugsziffernliste

- 1 .......................: Hose
- 2 .......................: Außenhülle
- 3 .......................: Bauchbund
- 4 .......................: Beinbund
- 5 .......................: Einsatz
- 6 .......................: Ringdüse
- 7 .......................: Noppe
- 8 .......................: Absaugschlauch
- 9 .......................: Zuführschlauch
- 9a .....................: Verteilungsschlauch
- 10 .....................: Zerkleinerungseinrichtung

## Patentansprüche

1. Vorrichtung zur hygienischen Entsorgung von humanen Ausscheidungsprodukten, welche eine Hose (1) aus einem gas- und flüssigkeitsdichten und elastischen Material mit einem Bauchbund (3) und Beinbünden (4) aufweist, wobei die Hose (1) mit zumindest einem mit einer Absaugeinrichtung verbindbaren Absaugschlauch (8) versehen ist oder mit einem solchen verbindbar ist,
dadurchgekennzeichnet,
dass die Hose (1) eine mit offenporigem Schaumstoff und/oder mit elastischen Noppen (7) bedeckte Innenseite sowie eine Einrichtung zum Einbringen von Flüssigkeit aufweist und wobei der Bauchbund (3) und die Beinbünde (4) innenseitige Einsätze (5) aus offenporigem Schaumstoff aufweisen.

2. Vorrichtung nach Anspruch 1, **dadurch gekennzeichnet, dass** die Einrichtung zum Einleiten von Flüssigkeiten Ringdüsen (6) aufweist, die in oder auf den Einsätzen (5) aus offenporigem Schaumstoff positioniert sind.

3. Vorrichtung nach Anspruch 1, **dadurch gekennzeichnet, dass** sie eine Einrichtung zum Einleiten von Luft oder Gas mittels weiterer im Bereich des Bauchbundes (3) und der Beinbünde (4) positionierter Ringdüsen aufweist, die außerhalb der zum Einleiten von Flüssigkeiten vorgesehen Ringdüsen (6) verlaufen.

4. Vorrichtung nach einem der Ansprüche 1 bis 3, **dadurch gekennzeichnet, dass** die Einrichtung zum Einleiten von Flüssigkeit und jene zum Einleiten von Luft oder Gas jeweils zumindest einen Zuführschlauch (9) und ggf. zumindest einen Verteilungsschlauch (9a) aufweisen.

5. Vorrichtung nach einem der Ansprüche 1 bis 4, **dadurch gekennzeichnet, dass** die aus offenporigem Schaumstoff bestehenden Einsätze (5) eine Breite von 1,0 cm bis 5,0 cm aufweisen.

6. Vorrichtung nach einem der Ansprüche 1 bis 5, **dadurch gekennzeichnet, dass** der offenporige Schaumstoff eine Dicke von 0,1 cm bis 5,0 cm aufweist.

7. Vorrichtung nach einem der Ansprüche 1 bis 6, **dadurch gekennzeichnet, dass** der offenporige Schaumstoff aus Polypropylen oder Polyethylen besteht.

8. Vorrichtung nach Anspruch 1, **dadurch gekennzeichnet, dass** die Noppen (7) aus dem Material der Hose (1) bestehen.

9. Vorrichtung nach Anspruch 1 oder 8, **dadurch gekennzeichnet, dass** die Noppen (7) eine Höhe von 1,0 cm bis 3,0 cm, insbesondere bis zu 1,0 cm aufweisen.

10. Vorrichtung nach einem der Ansprüche 1, 8 oder 9, **dadurch gekennzeichnet, dass** die Noppen (7) kegelförmig mit abgerundeter Spitze ausgeführt sind.

11. Vorrichtung nach einem der Ansprüche 1 bis 10, **dadurch gekennzeichnet, dass** die Hose (1) und die Noppen (7) aus einem Elastomer, insbesondere aus einem Gummimaterial oder aus Kunststoff, oder aus Silikon bestehen.

12. Vorrichtung nach einem der Ansprüche 1 bis 11, **dadurch gekennzeichnet, dass** sie eine Trennvorrichtung zum Trennen der im abgesaugten Gemisch enthaltenen Gase, Flüssigkeiten und Feststoffe aufweist.

13. Vorrichtung nach Anspruch 12, **dadurch gekennzeichnet, dass** sie einen Speicher zur Aufnahme der abgetrennten Flüssigkeit und/oder der abgetrennten Feststoffe aufweist.

14. Vorrichtung nach Anspruch 12, **dadurch gekennzeichnet, dass** sie eine Vorrichtung zum Aufbereiten des abgetrennten Gases zu dessen Wiederverwendung aufweist.

## Claims

1. Device for the hygienic disposal of human excretions, having pants (1) made from a gastight and liquid tight and elastic material with a waist band (3) and leg bands (4), wherein the pants (1) are provided with at least one extraction hose (8) connectable with an extraction device or connectable with something similar,
**characterised in that**
the pants (1) have an inner side coated with open-pore foam and/or with elastic nubs (7) and a device allowing entry of liquid and wherein the waist band (3) and the leg bands (4) have inserts (5) of open-pore foam on the inside.

2. Device according to claim 1, **characterised in that** the device for introducing liquids has ring nozzles (6), which are positioned in or on the inserts (5) of open-pore foam.

3. Device according to claim 1, **characterised in that** it has a device for inserting air or gas by means of other ring nozzles located in the region of the waist band (3) and the leg bands (4), which run outside the ring nozzles (6) provided for introducing liquids.

4. Device according to any one of claims 1 to 3, **characterised in that** the device for introducing liquid and the device for inserting air or gas has at least one feed hose (9) and, if necessary, at least one distribution hose (9a).

5. Device according to any one of claims 1 to 4, **characterised in that** the width of the inserts (5) consisting of open-pore foam is from 1.0 cm to 5.0 cm.

6. Device according to any one of claims 1 to 5, **characterised in that** the thickness of the open-pore foam is from 0.1 cm to 5.0 cm.

7. Device according to any one of claims 1 to 6, **characterised in that** the open-pore foam consists of polypropylene or polyethylene.

8. Device according to claim 1, **characterised in that** the nubs (7) are made in the same material as the pants (1).

9. Device according to claim 1 or 8, **characterised in that** the height of the nubs (7) is from 1.0 cm to 3.0 cm, in particular up to 1.0 cm.

10. Device according to any one of claims 1, 8 or 9, **characterised in that** the nubs (7) are made in the shape of a cone with a rounded tip.

11. Device according to any one of claims 1 to 10, **characterised in that** the pants (1) and the nubs (7) consist of an elastomer, in particular of a rubber material or of plastic, or of silicon.

12. Device according to any one of claims 1 to 11, **characterised in that** it has a separating device for separating the mixture of gases, liquids and solids sucked away.

13. Device according to claim 12, **characterised in that** it has a storage facility for accepting the separated liquid and/or the separated solids.

14. Device according to claim 12, **characterised in that** it has a device for treating the separated gases for reuse.

## Revendications

1. Dispositif pour l'élimination hygiénique d'excréments humains qui présente une culotte (1) fabriquée à partir d'un matériau élastique étanche aux gaz et aux liquides muni d'un tour ventral (3) et des tours de jambe (4), ladite culotte (1) présentant au moins un tuyau d'aspiration (8) pouvant être raccordé à un dispositif d'aspiration ou qui peut être raccordé à un appareil de ce type,
**caractérisé en ce que** la culotte (1) présente une face interne recouverte d'une mousse à alvéoles ouverts et/ou de protubérances élastiques (7) ainsi qu'un dispositif destiné à introduire du liquide et que le tour ventral (3) et les tours de jambe (4) présentent des inserts internes (5) en mousse à alvéoles ouverts.

2. Dispositif selon la revendication 1, **caractérisé en ce que** le dispositif destiné à introduire des liquides présente des buses annulaires (6) placées dans ou sur les inserts (5) en mousse à alvéoles ouverts.

3. Dispositif selon la revendication 1, **caractérisé en ce qu'**il présente un dispositif destiné à introduire de l'air ou du gaz au moyen d'autres buses annulaires placées dans la région du tour ventral (3) et des tours de jambe (4) et s'étendant à l'extérieur des buses annulaires (6) prévues pour l'introduction des liquides.

4. Dispositif selon l'une quelconque des revendications de 1 à 3, **caractérisé en ce que** le dispositif destiné à introduire du liquide et celui destiné à introduire de l'air ou du gaz présentent chacun au moins un tuyau d'alimentation (9) et, éventuellement, au moins un tuyau de distribution (9a).

5. Dispositif selon l'une quelconque des revendications de 1 à 4, **caractérisé en ce que** les inserts (5) fabriqués en mousse à alvéoles ouverts ont une largeur de 1,0 cm à 5,0 cm.

6. Dispositif selon l'une quelconque des revendications de 1 à 5, **caractérisé en ce que** la mousse à alvéoles ouverts a une largeur de 0,1 cm à 5,0 cm.

7. Dispositif selon l'une quelconque des revendications de 1 à 6, **caractérisé en ce que** la mousse à alvéoles ouverts est fabriquée à partir du polypropylène ou de polyéthylène.

8. Dispositif selon la revendication 1, **caractérisé en ce que** les protubérances (7) sont fabriquées à partir du matériau de la culotte (1).

9. Dispositif selon la revendication 1 ou 8, **caractérisé en ce que** les protubérances (7) ont une hauteur de 1,0 cm à 3,0 cm, en particulier jusqu'à 1,0 cm.

10. Dispositif selon l'une quelconque des revendications 1, 8 ou 9, **caractérisé en ce que** les protubérances (7) ont une forme conique avec le sommet arrondi.

11. Dispositif selon l'une quelconque des revendications de 1 à 10, **caractérisé en ce que** la culotte (1) et les protubérances (7) sont fabriquées à partir d'un élastomère, en particulier à partir d'un matériau en caoutchouc ou en matière plastique ou en silicone.

12. Dispositif selon l'une quelconque des revendications de 1 à 11, **caractérisé en ce qu'**il présente un dispositif de séparation destiné à séparer les gaz, les liquides et les matières solides contenus dans le mélange aspiré.

13. Dispositif selon la revendication 12, **caractérisé en ce qu'**il présente un réservoir destiné à recevoir le liquide séparé et/ou des matières solides séparées.

14. Dispositif selon la revendication 12, **caractérisé en ce qu'**il présente un dispositif destiné au traitement des gaz séparés pour leur réutilisation.
